# EUROPÄISCHE PATENTANMELDUNG

(11) **EP 4 796 027 A1**
(43) Veröffentlichungstag der Anmeldung: **26.08.2026**
(21) Anmeldenummer: 25159429.7
(22) Anmeldetag: 21.02.2025
(51) Int. Cl.: A61B 5/055, A61B 6/03, A61B 6/04, A61B 6/00, A61B 6/50, G06T 7/70

(54) **VERFAHREN ZUR POSITIONIERUNG EINES PATIENTEN, VERARBEITUNGSVORRICHTUNG, MAGNETRESONANZEINRICHTUNG, COMPUTERPROGRAMM UND DATENTRÄGER**

(71) Anmelder: Siemens Healthineers AG, 91301 Forchheim (DE)
(72) Erfinder: Wagner, Fabian, 91052 Erlangen (DE)
(74) Vertreter: Siemens Healthineers Patent Attorneys

(57) **Zusammenfassung**

Computerimplementiertes Verfahren zur Positionierung eines Patienten (1) bezüglich wenigstens einer Komponente (2) einer medizinischen Bildgebungseinrichtung (3) zur Erfassung von Ergebnisbilddaten (4), umfassend die Schritte:
- Erfassen vorläufiger Bilddaten (5), die wenigstens ein vorgegebenes anatomisches Merkmal (6) des Patienten (1) zumindest zum Teil abbilden, während der Patient (1) oder ein das anatomische Merkmal (6) umfassender Abschnitt des Patienten (1) durch eine Lagereinrichtung (7) der medizinischen Bildgebungseirichtung (3) gelagert ist,
- Ermitteln einer Merkmalsposition (8) des vorgegebenen anatomischen Merkmals (6) in den vorläufigen Bilddaten (5), und, stets oder bei Erfüllung einer von der Merkmalsposition (8) abhängigen Auslösebedingung (9),
- Ansteuern eines Aktors (10) der Bildgebungseinrichtung (3) zur Bewegung der Lagereinrichtung (7) in eine auf Basis der Merkmalsposition (8) ermittelte Sollposition (11) und/oder Ausgabe eines die Sollposition (11) betreffenden Hinweises (12) an einen Nutzer der Bildgebungseinrichtung (1).

## Beschreibung

Die Erfindung betrifft ein computerimplementiertes Verfahren zur Positionierung eines Patienten bezüglich wenigstens einer Komponente einer medizinischen Bildgebungseinrichtung zur Erfassung von Ergebnisbilddaten. Daneben betrifft die Erfindung ein computerimplementiertes Verfahren zum Bereitstellen eines durch Maschinenlernen trainierten Modells, eine Verarbeitungsvorrichtung, eine Magnetresonanzeinrichtung, ein Computerprogramm und einen Datenträger.

Magnetresonanztomographie (MRT) ist ein nicht-invasives bildgebendes Verfahren, das eine Visualisierung der inneren Anatomie und des Gewebes im menschlichen Körper ermöglicht. Insbesondere können auf Basis der Messdaten volumetrische 3D-Bildern rekonstruiert werden, die als Grundlage für diagnostische Entscheidungen im klinischen Arbeitsablauf dienen können. MRT-Aufnahmen werden in der Regel durch einen Arzt geplant, der die zu untersuchende anatomische Region vorgibt. Der Scan wird dann typischerweise durch medizinisches Fachpersonal durchgeführt. Hierbei erfolgt zunächst eine Positionierung des Patienten derart, dass die gewünschte anatomische Region abgebildet werden kann. Hierzu werden üblicherweise gut erkennbare Orientierungspunkte, wie Gelenke oder große anatomische Merkmale, zur groben Ausrichtung des Patienten verwendet. Um die Position der interessierenden Anatomie innerhalb des Sichtfeldes (englisch: field of view, FOV) der Magnetresonanzeinrichtung genau zu erfassen, wird ein schneller, niedrig auflösender Lokalisierungsscan mit großem Sichtfeld durchgeführt.

Die Positionierung des Patienten kann erschwert sein, wenn die genaue Position von relevanten Orientierungspunkten durch das medizinische Fachpersonal nicht erkennbar ist. Beispielsweise wird bei einer Bildgebung am Knie häufig eine um das Bein gelegte, flexible Lokalspule genutzt, die das Kniegelenk verdeckt. Aus diesem und anderen Gründen kann es zu einer Fehlpositionierung des Patienten kommen, wodurch die zu untersuchende anatomische Region unter Umständen im Lokalisierungsscan nicht vollständig abgebildet wird oder zumindest weit von einer Sollposition, als beispielsweise vom Iso-Zentrum der Bildgebung, entfernt ist.

Es kann daher erforderlich sein, die Patientenposition nach der Durchführung des Lokalisierungsscan durch ein manuelles Verstellen der Patientenliege zu korrigieren. Insbesondere bei einer großen Abweichung der Istposition des Patienten von einer Sollposition ist eine unmittelbare korrekte Abschätzung der erforderlichen Positionskorrektur durch das medizinische Fachpersonal häufig nicht möglich, sodass die Positionsanpassung im klinischen Alltag in vielen Fällen iterativ nach dem Prinzip von Versuch und Irrtum erfolgt.

Ein derartiges Vorgehen ist jedoch Zeit- und Arbeitsaufwändig und führte somit zur einer weniger effizienten Nutzung der Magnetresonanzeinrichtung und einer erhöhten Belastung für medizinisches Fachpersonal. Zudem ist zum Erreichen einer präzisen Positionierung, die typischerweise zum Erreichen einer hohen Bildqualität erforderlich ist, mit vertretbarem Zeitaufwand ein erhebliches Maß an Training und Erfahrung des medizinischen Fachpersonals erforderlich, sodass ein hoher Trainingsaufwand und lange Einarbeitungszeiten resultieren können.

Der Erfindung liegt die Aufgabe zugrunde, einen verbesserten Ansatz zur Positionierung eines Patienten bezüglich einer medizinischen Bildgebungseinrichtung anzugeben.

Die Aufgabe wird erfindungsgemäß durch ein Verfahren zur Positionierung eines Patienten bezüglich wenigstens einer Komponente einer medizinischen Bildgebungseinrichtung zur Erfassung von Ergebnisbilddaten gelöst, dass die folgenden Schritte umfasst:
- Erfassen vorläufiger Bilddaten, die wenigstens ein vorgegebenes anatomisches Merkmal des Patienten zumindest zum Teil abbilden, während der Patient oder ein das anatomische Merkmal umfassender Abschnitt des Patienten durch eine Lagereinrichtung der medizinischen Bildgebungseirichtung gelagert ist,
- Ermitteln einer Merkmalsposition des vorgegebenen anatomischen Merkmals in den vorläufigen Bilddaten, und, stets oder bei Erfüllung einer von der Merkmalsposition abhängigen Auslösebedingung,
- Ansteuern eines Aktors der Bildgebungseinrichtung zur Bewegung der Lagereinrichtung in eine auf Basis der Merkmalsposition ermittelte Sollposition und/oder Ausgabe eines die Sollposition betreffenden Hinweises an einen Nutzer der Bildgebungseinrichtung.

Im Rahmen der Erfindung wurde erkannt, dass für einen Positionierung des Patienten relevante anatomische Merkmale in vorläufigen Bilddaten, die bei medizinischen Bildgebungsmodalitäten häufig ohnehin vor der Hauptbildgebung erfasst werden, als beispielsweise beim eingangs genannten Lokalisierungsscan für eine Magnetresonanzaufnahme, typischerweise mit hinreichender Genauigkeit automatisiert lokalisierbar sind, um eine Abweichung ihrer Merkmalsposition von einer für die Hauptbildgebung gewünschten Sollmerkmalsposition zu ermitteln. Bezüglich der Positionierung des Patienten kann zudem in vielen Anwendungsfällen zumindest näherungsweise davon ausgegangen werden, dass die relevanten anatomischen Merkmale als ortsfest bezüglich der Lagereinrichtung angenommen werden können. Somit kann die Solposition der Lagereinrichtung beispielsweise als Sollrelativposition zu einer während der Erfassung der vorläufigen Bilddaten vorliegenden Istposition der Lagereinrichtung ermittelt werden, die einer Abweichung der Merkmalsposition von einer vorgegeben Sollmerkmalsposition entspricht. Häufig ist in medizinischen Bildgebungseinrichtungen jedoch auch eine Absolutposition der Lagereinrichtung während der Ermittlung der vorläufigen Bilddaten bekannt, sodass die Sollposition auch als Absolutposition bestimmt werden kann.

Es ist prinzipiell möglich, dass die Sollposition den Ort und/oder die Orientierung der Lagereinrichtung absolut oder relativ zu der Lage während der Erfassung der vorläufigen Bilddaten vollständig definiert, also insbesondere bezüglich aller drei Bewegungs- und/oder Rotationsfreiheitsgrade. Je nach Bewegungsfreiheitsgraden des Aktors beziehungsweise der Lagereinrichtung und/oder je nachdem, welche Art von anatomischem Merkmal betrachtet wird, kann es jedoch zweckmäßig sein, dass die Sollposition nur eine Absolutposition oder Relativposition für einen oder zwei Bewegungs- und/oder Rotationsfreiheitsgrade vorgibt. Beispielsweise kann eine als Lagereinrichtung genutzte Patienten liege ausschließlich in die Längsrichtung und/oder eine Querrichtung verschiebbar sein.

Der die Sollposition betreffende Hinweis kann dazu dienen, einen Nutzer bei einer manuellen Verstellung der Lagereinrichtung zu unterstützen. Beispielsweise kann der Nutzer durch den Hinweis aufgefordert werden, die Lagereinrichtung eine vorgegebene Weglänge weit in eine in dem Hinweis angegebene Richtung zu verschieben und/oder um einen vorgegebenen Winkel um eine vorgegebene Achse zur verschwenken.

Die Auslösebedingung kann insbesondere erfüllt werden oder nur dann erfüllbar sein, wenn die Abweichung der ermittelten Merkmalsposition von einer vorgegebenen Istmerkmalsposition einen vorgegebenen Grenzwert überschreitet. Hierbei kann im einfachsten Fall ausschließlich der Betrag der Abweichung berücksichtigt werden, es ist jedoch auch möglich, für verschiedene Richtungen der Abweichungen verschiedene Grenzwerte vorzugeben oder Ähnliches.

Es ist möglich, dass ausschließlich die Ansteuerung des Aktors ausschließlich bei Erfüllung der Auslösebedingung erfolgt, während die Gabe des die Sollposition betreffenden Hinweises stets oder bereits bei Erfüllung einer Teilbedingung der Auslösebedingung oder auch bei Nichterfüllung der Auslösebedingung erfolgt. Beispielsweise kann als der die Sollposition betreffende Hinweis bei Nichterfüllung der Auslösebedingung ein Hinweis ausgegeben werden, dass der Patient beziehungsweise die Lagerreinrichtung bereits korrekte beziehungsweise an der Sollposition positioniert ist.

Die Komponente, bezüglich der der Patient positioniert werden soll, kann insbesondere eine unmittelbar an der Bildgebung beziehungsweise an der Erfassung von der Bildgebung zugrunde liegenden Daten beteiligt sein. Wird als Bildgebungseinrichtung eine Magnetresonanzeinrichtung genutzt, kann die Positionierung insbesondere bezüglich wenigstens einer Empfangsspule und/oder bezüglich wenigstens einer Sendespule und/oder bezüglich wenigstens einer Feldspule erfolgen. Bei üblichen Magnetresonanzeinrichtung sind die genannten Spulen typischerweise zumindest zum Teil ortsfest bezüglich eines Freiraums für den Patienten angeordnet, sodass die Positionierung bezüglich dieses Freiraums erfolgen kann. Bei einer Röntgenbildgebung kann die Positionierung hingegen insbesondere bezüglich einer Röntgenröhre und/oder eines Röntgendetektors erfolgen. Bei anderen Bildgebungsmodalitäten, beispielsweise bei einer Ultraschallbildgebung und/oder bei einer molekularen Bildgebung, kann die Positionierung insbesondere bezüglich jeweils wenigstens eines im Rahmen der Bilddatenerfassung genutzten Sensors erfolgen.

Die Lagereinrichtung kann insbesondere eine Patientenliege sein, jedoch beispielsweis auch ein Sitz oder eine Stützfläche für eine Gliedmaße des Patienten, die das anatomische Merkmal umfasst. Die Position des Patienten und der Lagereinrichtung und die Merkmalsposition können jeweils einen Ort und/oder eine Orientierung des Patienten beziehungsweise der Lagereinrichtung beziehungsweise des Merkmals beschreiben.

Durch einen Segmentierungsalgorithmus kann eine Segmentierung wenigstens eines Knochens des Patienten in den vorläufigen Bilddaten erfolgen, wobei die Merkmalsposition in Abhängigkeit der Segmentierung ermittelt wird. Insbesondere große Knochenstrukturen sind gut geeignet, eine Positionierung des Patienten bezüglich eines Bildgebungsbereichs und somit bezüglich der bildgebungsrelevanten Komponenten der Bildgebungseinrichtung zu definieren. Hierdurch kann beispielsweise ähnlich wie bei einer manuellen Positionierung auf die Positionierung wenigstens eines Gelenks des Patienten im Bildgebungsbereich abgestellt werden, sodass Erfahrungswerte aus der bislang üblichen manuellen Patientenpositionierung zur Vorgabe der Sollmerkmalsposition genutzt werden können.

Eine Segmentierung wenigstens eines Knochens als anatomisches Merkmal oder zur Definition des anatomischen Merkmals ist auch bei einer Magnetresonanzbildgebung zweckmäßig, obwohl übliche Magnetresonanzsequenzen nicht auf hohe Kontraste bei der Abbildung von Knochen optimiert sind. Typischerweise werden zumindest für vorbereitende Lokalisierungsscans Messsequenzen genutzt, die hinreichend hohe Knochenkontraste bereitstellen, da beispielsweise die Positionen von Gelenken, wie bereits eingangs erläutert wurde, auch bei einer manuellen Positionierung des Patienten und auch bei einer Planung von im Rahmen der Magnetresonanzbildgebung abzutastenden Schichten hochrelevant sind.

Der Knochen beziehungsweise das den Knochen abbildende Segment kann unmittelbar als das anatomische Merkmal genutzt werden, dessen Merkmalsposition bestimmt wird. Da jedoch größere Knochen, wie beispielsweise das Waden- und Schienbein bei der Bildung eines Knies, in typischerweise zur Lokalisierung genutzten vorläufigen Bilddaten häufig nicht vollständig abgebildet sind, kann es zweckmäßig sein, als anatomisches Merkmal einen bestimmten Teil des Knochens, also beispielsweise ein proximales oder distales Ende des Knochens heranzuziehen.

Als besonders zweckmäßig hat es sich jedoch herausgestellt, zwischen Knochen verbleibende Spalte beziehungsweise Grenzflächen zwischen Knochen als anatomisches Merkmal zu lokalisieren. Daher kann für zwei der Knochen, die benachbart zueinander angeordnet sind, ein jeweiliges den jeweiligen Knochen abbildendes Segment der vorläufige Bilddaten ermittelt werden, wobei als das anatomische Merkmal, dessen Merkmalsposition bestimmt wird, ein zwischen diesen Segmenten angeordneter Zwischenbereich oder eine Grenzlinie oder Grenzfläche zwischen diesen Segmenten verwendet wird.

Als der Segmentierungsalgorithmus kann insbesondere ein durch Maschinenlernen trainiertes Modell verwendet werden. Im Bereich der Segmentierung von anatomischen Merkmalen beziehungsweise von medizinischen Bilddaten ist durch Algorithmen des Maschinenlernens eine besonders robuste Segmentierung und insbesondere eine robuste Zuordnung von Segmenten zu bestimmten anatomischen Merkmalen, also im erfindungsgemäßen Verfahren insbesondere zu Knochen, möglich. Somit ist auch dann eine robuste Segmentierung und Klassifikation möglich, wenn die segmentierten anatomischen Merkmale nur teilweise abgebildet sind und/oder wenn die Bildqualität, also beispielsweise eine Bildauflösung und/oder ein Signal-zu-Rauschverhältnis, der vorläufigen Bilddaten relativ gering ist, beispielsweise wenn ein Lokalisierungsscan mit kurzer Scanzeit genutzt wird.

Das durch Maschinenlernen trainierte Modell kann insbesondere unmittelbar die vorläufigen Bilddaten als Eingangsdaten verarbeiten. Alternativ wäre jedoch auch eine Vorverarbeitung der vorläufigen Bilddaten zur Bereitstellung der Eingangsdaten möglich. Das Training des Modells erfolgt bevorzugt außerhalb des beanspruchten Verfahrens und kann beispielsweise an einem anderen Ort und/oder durch andere Personen durchgeführt werden als das Verfahren zur Positionierung des Patienten. Beispielsweise kann das Training durch einen Hersteller der medizinischen Bildgebungseinrichtung erfolgen, während das Verfahren zur Positionierung des Patienten bei einem an Endanwender, also beispielsweise in einem Krankenhaus oder einer Arztpraxis, angewendet wird.

Im Allgemeinen bildet ein durch Maschinenlernen trainiertes Modell kognitive Funktionen ab, die Menschen mit anderen menschlichen Gehirnen assoziieren. Durch Training basierend auf Trainingsdaten (Maschinenlernen) ist die trainierte Funktion in der Lage, sich an neue Umstände anzupassen und Muster zu detektieren und zu extrapolieren. Ein anderer Ausdruck für "ein durch Maschinenlernen trainiertes Modell" ist "eine trainierte Funktion".

Allgemein gesagt können Parameter eines trainierten Modells durch Training angepasst werden. Insbesondere können überwachtes Lernen, halbüberwachtes Lernen, nicht überwachtes Lernen, Reinforcement Learning und/oder aktives Lernen verwendet werden. Darüber hinaus kann auch Repräsentationslernen (auch als "feature learning" bekannt) eingesetzt werden. Die Parameter der trainierten Funktion können insbesondere iterativ durch mehrere Trainingsschritte angepasst werden. Insbesondere kann bei dem Training eine bestimmte Kostenfunktion minimiert werden. Beispielsweise kann beim Training eines neuronalen Netzes der Backpropagation-Algorithmus eingesetzt werden.

Eine trainierte Funktion kann beispielsweise ein neuronales Netz, ein Transformer, eine Support Vector Machine (SVM), einen Entscheidungsbaum und/oder ein Bayes-Netzwerk umfassen und/oder die trainierte Funktion kann auf k-means-Clustering, Q-Learning, genetischen Algorithmen und/oder Zuordnungsregeln basieren. Insbesondere kann ein neuronales Netzwerk ein tiefes neuronales Netzwerk, ein Convolutional Neural Network (CNN) oder ein tiefes CNN sein. Darüber hinaus kann das neuronale Netzwerk ein Adversarial Network, ein tiefes Adversarial Network und/oder ein Generative Adversarial Network (GAN) sein.

Das durch Maschinenlernen trainierte Modell kann insbesondere ein neuronales Netz mit einer U-Net-Struktur sein oder umfassen. Ein neuronales Netz mit U-Net-Struktur hat eine Encoder-Decoder-Struktur, die besonders gut zur Segmentierung auch feiner Strukturen geeignet ist. Die grundlegende Netzwerkarchitektur wurde erstmals in O. Ronneberger et al., "U-Net: Convolutional Networks for Biomedical Image Segmentation", arXiv:1505. 04597v1, beschrieben. Beispielsweise kann das quelloffene nnU-Net (https://github.com/MIC-DKFZ/nnUNet, vgl. auch Isensee, F., et al. nnU-Net: a self-configuring method for deep learning-based biomedical image segmentation. Nat Methods 18, 203-211 (2021). https://doi.org/10.1038/s41592-020-01008-z) genutzt werden.

Das trainierte Modell kann beispielsweise durch ein überwachtes Training auf Basis des nnU-Net oder eines anderen zu trainierenden Modells bereitgestellt werden. Als geeignete Trainingsdatensätze können beispielsweise Trainings-Bilddaten genutzt werden, die beispielsweise manuell segmentiert und klassifiziert sind, um den einzelnen Bildpunkten der jeweiligen Trainings-Bilddaten eine Klasse zuzuordnen, die das dort abgebildete anatomische Merkmal klassifiziert.

Um für den Nutzer, also insbesondere für medizinisches Personal, unerwartete Bewegung der Lagereinrichtung und somit des Patienten zu vermeiden, kann es zweckmäßig sein, die Ansteuerung des Aktors erst nach einer nutzerseitigen Freigabe durchzuführen. Es ist daher möglich, dass bei Erfüllung einer die Sollposition auswertenden ersten Teilbedingung der Auslösebedingung zunächst eine Freigabeanforderung an den Nutzer ausgegeben wird, wobei die Ansteuerung des Aktors zur Bewegung der Lagereinrichtung in die Sollposition nur bei Erfüllung einer zweiten Teilbedingung der Auslösebedingung erfolgt, wobei die zweite Teilbedingung erfüllt wird oder ausschließlichen dann erfüllbar ist, wenn eine eine Freigabe der Bewegung indizierenden Bedieneingabe des Nutzers an der medizinischen Bildgebungseinrichtung und/oder an einer in einer Kommunikationsverbindung mit der medizinischen Bildgebungseinrichtung stehenden Freigabeeinrichtung erfasst wird.

Die Freigabeanforderung kann der die Sollposition betreffende Hinweis sein oder diesen umfassen. Beispielsweise kann es dem Nutzer hierbei auch ermöglicht werden, durch verschiedene Bedieneingaben zwischen verschiedenen Optionen, beispielsweise zwischen der automatischen Positionierung der Lagereinrichtung und/oder einer manuell durchgeführten oder durch Benutzereingaben gesteuerten aktorischen Verstellung der Lagereinrichtung und/oder einer Positionierung des Patienten auf der Lagereinrichtung mit anschließender Neupositionierung der Lagereinrichtung, zu wählen.

Der die Sollposition betreffende Hinweis und/oder die Freigabeanforderung kann eine Erläuterungsinformation für den Nutzer umfassen, weshalb eine Bewegung der Lagereinrichtung zweckmäßig erscheint. Die Erläuterungsinformation kann beispielsweise ein Text oder eine Sprachnachricht sein. Im einfachsten Fall kann die Erläuterungsinformation für die medizinische Bildgebungseinrichtung oder bevorzugt für ein jeweiliges Bildgebungsaufgabe, beispielsweise zur Abbildung eines bestimmten anatomischen Merkmals, fest vorgegeben sein.

Bevorzug kann jedoch stets oder bei Erfüllung der Auslösebedingung und/oder der ersten Teilbedingung in Abhängigkeit der Merkmalsposition eine von mehreren vorgegebenen Erläuterungsinformationen ausgewählt und als Teil des die Sollposition betreffenden Hinweises und/oder der Freigabeanforderung ausgegeben werden.

Die jeweilige Erläuterungsinformation kann insbesondere eine Begründung sein oder umfassen, weshalb eine Änderung der Position der Lagereinrichtung und somit des Patienten für die aktuelle Bildgebungsaufgabe zweckmäßig ist. Durch Berücksichtigung der Merkmalsposition bei der Auswahl der Erläuterungsinformation kann beispielsweise zwischen Fällen unterschieden werden, in denen in der aktuellen Position des Patienten ein typischerweise bei der aktuellen Bildgebungsaufgabe relevantes anatomisches Merkmal unnötig weit von einem Isozentrum der Bildgebung beabstandet sein wird, wodurch die Bildqualität reduziert sein kann, und Fällen bei denen beispielsweise eines von mehreren potentiell relevanten anatomischen Merkmalen unter Umständen nicht vollständig abgebildet werden kann. Hierdurch kann es dem Benutzer ermöglicht werden, selbst zu beurteilen, ob der angegebene Grund für die tatsächliche vorliegende Bildgebungsaufgabe seines Erachtens relevant ist oder nicht. Auf dieser Basis kann beispielsweise eine nutzerseitige Entscheidung getroffen werden, ob weitere Zeit für eine Umpositionierung aufgewendet werden soll oder ob unmittelbar eine Bildgebung erfolgen soll.

Die Zuordnung der Erläuterungsinformationen zu den Merkmalspositionen kann beispielsweise durch eine Look-Up-Tabelle oder Ähnliches erfolgen. Beispielsweise können durch einen oder mehrere Experten im Rahmen der Bereitstellung eines das Verfahren implementierenden Programms verschiedenen Werten oder Wertebereichen der Merkmalsposition und/oder Werten oder Wertebereichen einzelner Koordinaten der Merkmalsposition unterschiedliche Erläuterungsinformationen zugeordnet werden.

Die Erfassung der vorläufiger Bilddaten kann nach der Ansteuerung des Aktors zur Bewegung der Lagereinrichtung in die Sollposition automatisch zur Ermittlung neuer vorläufiger Bilddaten wiederholt werden. Auf Basis der neuen vorläufiger Bilddaten kann dann beispielsweise eine neue Merkmalsposition des vorgegebenen anatomischen Merkmals ermittelt werden, um beispielsweise zu validieren, dass diese nun einer Sollmerkmalsposition entspricht beziehungsweise dass eine Abweichung von der Sollmerkmalsposition nun unterhalb eines vorgegebenen Grenzwertes liegt. Ist dies nicht der Fall, so kann beispielsweise auf Basis der neuen Merkmalsposition eine neue Sollposition ermittelt und zur erneuten Bewegung der Lagereinrichtung mittels des Aktors genutzt werden und/oder ein Hinweis bezüglich der fehlerhaften Positionierung an den Nutzer ausgegeben werden.

Ergänzend oder alternativ können die neuen vorläufiger Bilddaten zu einer Planung der Hauptbildgebung, die zur Erfassung der Ergebnisbilddaten dient, genutzt werden. Beispielsweise kann bei Nutzung einer Magnetresonanzeinrichtung als medizinische Bildgebungseinrichtung eine Planung von Bildgebungsschichten auf Basis der neuen vorläufigen Bilddaten erfolgen.

Als medizinischen Bildgebungseinrichtung kann insbesondere eine Magnetresonanzeinrichtung verwendet werden. Wie bereits eingangs erläutert, kann die Positionierung eines Patienten bei einer Magnetresonanzbildgebung zusätzlich dadurch erschwert sein, dass wenigstens ein relevantes anatomisches Merkmal, beispielsweise ein Gelenk, durch eine Lokalspule verdeckt ist. Daher ist das erfindungsgemäße Verfahren bei dieser Bildgebungsmodalität besonders relevant. Zudem wird bei einer Magnetresonanzbildgebung häufig ohnehin ein Lokalisierungsscan zur Validierung der Patientenposition und/oder zur Schichtenplanung genutzt, sodass das erfindungsgemäße Vorgehen ohne weiteres in den klinischen Alltag beziehungsweise das übliche Vorgehen zur Bildgebung integriert werden kann, wodurch, wie bereits erläutert, der Zeit- und Arbeitsaufwand zur Bilddatenerfassung erheblich reduzieren kann.

Die Erfindung betrifft zudem ein computerimplementiertes Verfahren zum Bereitstellen eines durch Maschinenlernen trainierten Modells, das dazu eingerichtet ist, in dem erfindungsgemäßen computerimplementierten Verfahren zur Positionierung eines Patienten als der Segmentierungsalgorithmus und/oder zur Ermittlung der Merkmalsposition verwendet zu werden, umfassend die Schritte:
- Empfangen von Eingangs-Trainingsdaten,
- Empfangen von Ausgangs-Trainingsdaten, wobei die Ausgangs-Trainingsdaten eine jeweilige Klasse der Pixel oder Voxel der Eingangs-Trainingsdaten vorgeben und/oder eine Segmentierung der Eingangs-Trainingsdaten beschreiben,
- Trainieren Modells durch Maschinenlernen auf der Grundlage der Eingangs-Trainingsdaten und der Ausgangs-Trainingsdaten, und
- Bereitstellen des durch Maschinenlernen trainierten Modells.

Insbesondere ist in den Eingangs-Trainingsdaten zumindest ein vorgegebener Knochen segmentiert oder es ist eine Gruppe von Pixeln oder Voxeln als der vorgegebene Knochen klassifiziert. Insbesondere sind jeweils wenigstens zwei Knochen segmentiert beziehungsweise klassifiziert.

Wie bereits obig erläutert, kann somit ein an sich bekanntes überwachtes Lernen genutzt werden, bei dem insbesondere eine Kostenfunktion minimiert werden kann, die von einem Abstandmaß zwischen den auf Basis der Eingangs-Trainingsdaten durch das Modell ermittelten Ausgangsdaten und den Ausgangs-Trainingsdaten abhängt. Die Parameter des trainierten Modells können dann iterativ angepasst werden, um die Kostenfunktion für einen jeweiligen Batch von Trainingsdaten zu minimieren. Beispielsweise kann hierzu eine Fehlerrückführung, insbesondere mittels des Gradientenabstiegsverfahrens, genutzt werden.

Die Erfindung betrifft zudem eine Verarbeitungseinrichtung, die zur Durchführung des erfindungsgemäßen computerimplementierten Verfahrens zur Positionierung eines Patienten und/oder zur Durchführung des erfindungsgemäßen computerimplementierten Verfahrens zum Bereitstellen eines durch Maschinenlernen trainierten Modells eingerichtet ist. Die Verarbeitungseinrichtung kann beispielsweise als geeignet programmierte Datenverarbeitungsvorrichtungen ausgebildet sein oder die genannte Funktionalität kann alter-nativ zumindest zum Teil fest verdrahtet implementiert sein. Die Verarbeitungseinrichtung kann in eine medizinische Bildgebungseinrichtung integriert sein oder separat von dieser ausgebildet sein. Sie kann beispielsweise als Arbeitsplatzrechner, Server oder Cloudlösung implementiert sein.

Die Erfindung betrifft zudem eine Magnetresonanzeinrichtung, die eine erfindungsgemäße Verarbeitungseinrichtung umfasst. Durch die Integration der erfindungsgemäßen Verarbeitungseinrichtung und somit die Implementierung des erfindungsgemäßen Verfahrens in einer Magnetresonanzeinrichtung kann die Positionierung des Patienten besonders nahtlos in den Bildgebungsprozess integriert werden.

Die Erfindung betrifft zudem ein Computerprogramm mit Anweisungen, die dazu eingerichtet sind, bei einer Ausführung auf einer Datenverarbeitungsvorrichtung das erfindungsgemäße computerimplementierte Verfahren zur Positionierung eines Patienten und/oder zum Bereitstellen eines durch Maschinenlernen trainierten Modells durchzuführen.

Zudem betrifft die Erfindung einen Datenträger, der das erfindungsgemäße Computerprogramm umfasst.

*Unabhängig vom grammatikalischen Geschlecht eines bestimmten Begriffes sind Personen mit männlicher, weiblicher oder anderer Geschlechteridentität mit umfasst.*

Weitere Vorteile und Einzelheiten der Erfindung ergeben sich aus den folgen Ausführungsbeispielen sowie den zugehörigen Zeichnungen. Hierbei zeigen schematisch:
- Fig. 1: ein Ausführungsbeispiel der erfindungsgemäßen Magnetresonanzeinrichtung, die ein Ausführungsbeispiel der erfindungsgemäßen Verarbeitungseinrichtung umfasst,
- Fig. 2: ein Ablaufdiagramm eines Ausführungsbeispiels des erfindungsgemäßen Verfahrens zur Positionierung eines Patienten,
- Fig. 3: beispielhafte in dem Verfahren gemäß Fig. 2 ausgewertete vorläufige Bilddaten,
- Fig. 4: ein Ablaufdiagramm eines Ausführungsbeispiels des erfindungsgemäßen Verfahrens zum Bereitstellen eines durch Maschinenlernen trainierten Modells, und
- Fig. 5: eine beispielhafte Struktur eines durch Maschinenlernen trainierten beziehungsweise trainierbaren Modells, das in den Ausführungsbeispielen gemäß Fig, 2 und Fig. 4 als Segmentierungsalgorithmus beziehungsweise als zu trainierendes Modell nutzbar ist.

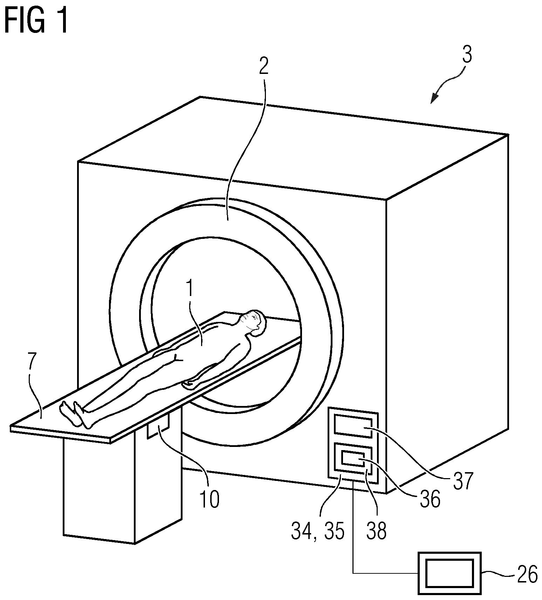

Fig. 1 zeigt eine medizinischen Bildgebungseinrichtung 3, die im Beispiel eine Magnetresonanzeinrichtung ist und in der gezeigten Betriebssituationen zur Erfassung von einen Patienten 1 betreffenden Ergebnisbilddaten 4 genutzt werden soll.

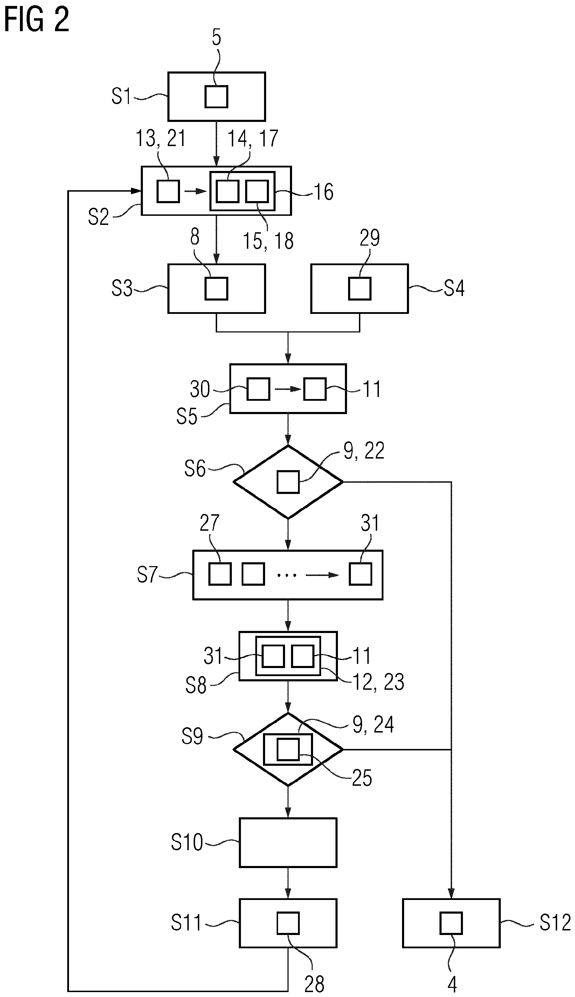

Ein nicht gezeigter Nutzer, also beispielsweise medizinisches Fachpersonal, soll bei der Positionierung des Patienten 1 bezüglich einer Komponente 2 der Magnetresonanzeinrichtung 3, also beispielsweise bezüglich des Patientenaufnahmeraums, unterstützt werden. Hierzu umfasst die Magnetresonanzeinrichtung 3 eine Verarbeitungseinrichtung 34, die dazu eingerichtet ist, auf Basis von vorläufigen Bilddaten 5 des Patienten 1 automatisch eine Sollposition 11 für eine den Patienten 1 tragende Lagereinrichtung 7 zu bestimmen. Im Beispiel kann diese Sollposition 11 dann, nach einer Freigabe durch den Nutzer, automatisch mittels des Aktors 10 der medizinischen Bildgebungseinrichtung 3 eingestellt werden. Ein beispielhaftes hierfür genutztes Verfahren zur Positionierung des Patienten 1 wird später noch mit Bezug auf Fig. 2 genauer erläutert werden.

Die Implementierung des Verfahrens erfolgt im Beispiel durch ein Computerprogramm 36, dass in einem Speicher 38 einer Datenverarbeitungsvorrichtung 35 der medizinischen Bildgebungseinrichtung 3 gespeichert ist und durch den dortigen Prozessor 37 ausgeführt wird. Alternativ wäre es beispielsweise möglich, das Verfahren durch eine externe Verarbeitungseinrichtung, beispielsweise auf einem Arbeitsplatzrechner oder einem Server, zu implementieren.

In dem in Fig. 2 gezeigten Ablaufdiagramm des Verfahrens zur Positionierung des Patienten 1 werden zunächst in Schritte S1 vorläufige Bilddaten 5 erfasst, die wenigstens ein vorgegebenes anatomisches Merkmal 6 des Patienten 1 zumindest zum Teil abbilden. Die vorläufigen Bilddaten 5 können beispielsweise durch einen im Bereich der Magnetresonanzbildgebung üblichen Lokalisierungsscan erfasst werden.

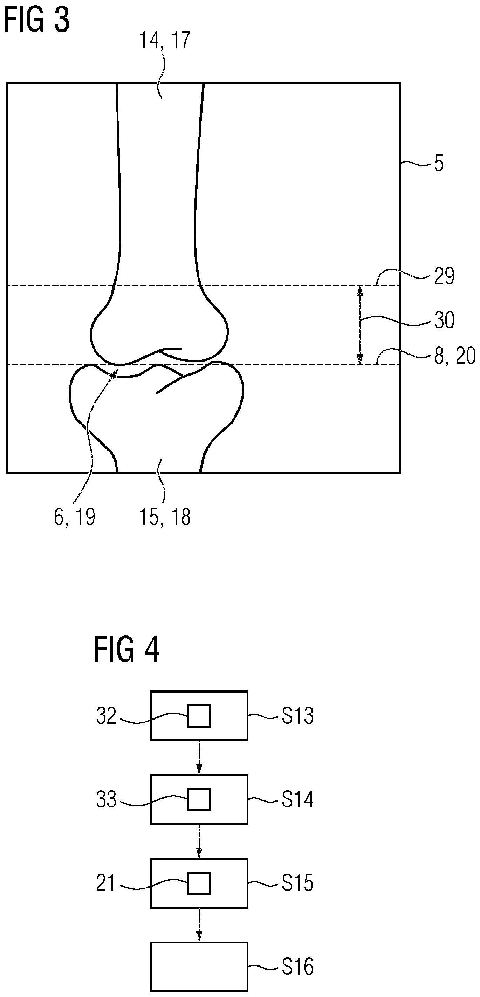

Ein Beispiel für vorläufige Bilddaten 5, die den Bereich eines Knies des Patienten 1 betreffen, ist in Fig. 3 dargestellt. Als anatomisches Merkmal 6 wird im Beispiel ein Zwischenbereich 19 zwischen zwei Knochen 14, 15, nämlich zwischen Schienbein und Wadenbein, betrachtet.

In Schritte S2 werden die vorläufigen Bilddaten 5 dann durch einen Segmentierungsalgorithmus 13 segmentiert. Die Segmentierung 16 erfolgt im Beispiel derart, dass für zwei benachbarte Knochen 14, 15, also im Bespiel für das Schien- und Wadenbein, ein jeweiliges den jeweiligen Knochen 14, 15 abbildendes Segment 17, 18 der vorläufige Bilddaten 5 ermittelt wird.

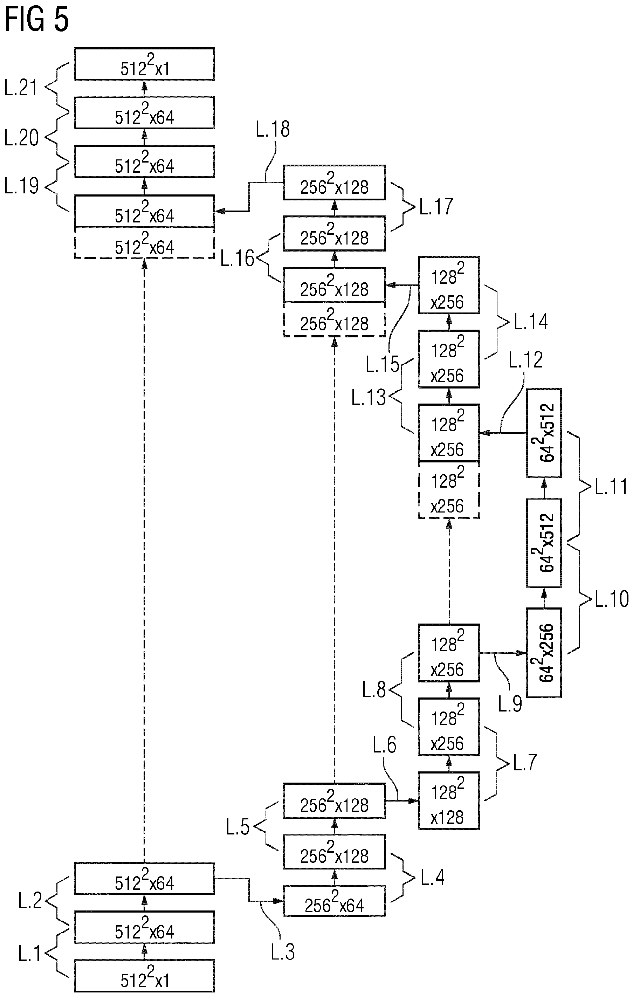

Als Segmentierungsalgorithmus 13 dient im Beispiel ein durch Maschinenlernen trainiertes Modell 21. Das trainierte Modell umfasst beispielswiese ein neuronales Netz oder auch eine Kaskade mehrerer neuronaler Netze mit U-Net-Struktur. Die grundsätzliche Struktur eines neuronalen Netzes mit U-Net-Struktur wird später noch mit Bezug auf Fig. 5 erläutert werden. Beispielsweise kann ein geeignet trainiertes Modell auf Basis des bereits eingangs genannten, quelloffenen nnU-Net genutzt werden. Ein mögliches Training wird später noch mit Bezug auf Fig. 4 erläutert werden.

In Schritt S3 wir auf Basis der Segmentierung 16 die Merkmalsposition 8 des anatomischen Merkmals 6 ermittelt. Im Beispiel wird hierbei davon ausgegangen, dass die Lagereinrichtung 7 durch den Aktor 10 ausschließlich in die Längsrichtung des Patienten 1 verschoben werden soll, sodass auch nur eine Sollposition 11 der Lagereinrichtung 7 für diese Richtung bestimmt werden soll. Daher kann als Merkmalsposition 8 die Positionen der Grenzfläche 20 zwischen den Segmenten 17, 18 verwendet werden.

Würde in einer Erweiterung des Beispiels beispielsweise auch eine automatische Verschiebung in Querrichtung des Patienten 1 gewünscht, könnte der Zwischenbereich 19 zusätzlich in Querrichtung in Fig. 3 lokalisiert werden oder es könnte ergänzend oder alternativ ein weiteres anatomisches Merkmal 6 ausgewertet werden, beispielsweise die Lage einer Mittelgeraden eines der Knochen 14, 15 und somit eines der Segment 17, 18.

Im Schritt S4 wird zudem eine Sollmerkmalsposition 29 vorgegeben. Diese kann beispielsweise durch das das Verfahren implementierende Computerprogramm 36 implizit oder explizit vorgegeben sein oder auch nutzerseitige einstellbar oder in Abhängigkeit einer eingestellten Bildgebungsaufgabe automatische wählbar sein. Im Beispiel befindet sich die Sollmerkmalsposition 29, an der der Zwischenbereich 19 beziehungsweise die Grenzfläche 20 angeordnet sein sollte, im Mittelbereich in Hochrichtung in Fig. 3, die der Längsrichtung beziehungsweise der Proximal-Distal-Richtung des Patienten 1 entspricht, wie auch in Fig. 3 schematisch dargestellt ist.

Im Schritt S5 wird dann auf Basis der Abweichung 30 zwischen der ermittelten Merkmalsposition 8 und der Sollmerkmalsposition 29 eine Sollposition 11 für die Lagereinrichtung 7 ermittelt. Die Sollposition 11 kann insbesondere eine Sollrelativposition der Lagereinrichtung 7 bezüglich der bei der Erfassung der vorläufigen Bilddaten 5 vorliegenden Istposition sein. Die Richtung, in der die Sollrelativposition bezüglich der Istpositionen liegt, ergibt sich dann daraus, ob die Merkmalsposition 8 in Fig. 3 oberhalb und unterhalb der Sollmerkmalsposition 29 liegt. Die Entfernung der Sollposition 11 von der Istposition ist durch die Abweichung 30 vorgegeben.

Im Schritt S6 wird dann eine erste Teilbedingung 22 einer Auslösebedingung 9 geprüft, wobei eine automatische Verschiebung der Lagereinrichtung 7 durch den Aktor 10 im Beispiel nur dann erfolgen soll, wenn sowohl die erste Teilbedingung 22 als auch eine später noch diskutierte zweite Teilbedingung 24 der Auslösebedingung 9 erfüllt sind. Die erste Teilbedingung 22 wird im Beispiel dann erfüllt, wenn die Sollposition 11 von der aktuellen Istposition der Lagereinrichtung 7 zumindest um einen vorgegebenen Grenzwert abweicht.

Ist dies der Fall, so wird in Schritt S7 im Beispiel in Abhängigkeit der Merkmalsposition 8 zunächst eine von mehreren vorgegebenen Erläuterungsinformationen 27 als ausgewählte Erläuterungsinformation 31 ausgewählt, die dann in Schritt S8 als Teil eines die Sollposition 11 betreffenden Hinweises 12 an den Nutzer ausgegeben wird. Im Beispiel erfolgt die Hinweisgabe über eine separat von der medizinischen Bildgebungseinrichtung 3 ausgebildete, mit dieser in einer Kommunikationsverbindung stehende Freigabeeinrichtung 26, die in Fig. 1 beispielhaft als Touchscreen dargestellt ist.

Die vorgegebenen Erläuterungsinformationen 27 können verschiedene Gründe angeben, weshalb auf Basis der ermittelten Merkmalsposition 8 eine Umpositionierung des Patienten 1 durch Bewegung der Lagereinrichtung 7 zweckmäßig erscheint. In dem Beispiel, für das in Fig. 3 vorläufige Bilddaten 5 gezeigt sind, soll ein Knie des Patienten 1 abgebildet werden. Je nach ermittelter Merkmalsposition 8 kann als ausgewählte Erläuterungsinformation in diesem Fall beispielsweise ausgegeben werden, dass in der aktuellen Patientenposition das Knie voraussichtlich nicht vollständig abgebildet werden kann oder dass aufgrund des großen Abstands des Knies von einem Isozentrum der medizinischen Bildgebungseinrichtung die Bildqualität beeinträchtigt sein könnte oder dass eine automatische Positionskorrektur aufgrund des sehr großen Positionsfehlers nicht möglich oder nicht zweckmäßig ist.

Da der Hinweis 12 im Beispiel die Sollposition 11 umfasst, wäre es prinzipiell möglich, dass der Nutzer auf Basis der Sollposition 11 die Position der Lagereinrichtung 11 oder unmittelbar des Patienten 1 selbst korrigiert. Da im Beispiel jedoch eine automatische Positionskorrektur mittels des Aktors 10 ermöglicht werden soll, dienten der Hinweis 12 im Beispiel gleichzeitig als Freigabeanforderung 23, durch die der Nutzer aufgefordert wird, eine eine Freigabe der Bewegung indizierenden Bedieneingabe 25 zu tätigen. Im Beispiel kann diese Bedieneingabe 25 mittels der Freigabeeinrichtung 26 erfasst werden, also beispielsweise auch beabstandet von der medizinischen Bildgebungseinrichtung 3, beispielsweise in einem Kontrollraum.

In Schritt S9. wird anschließend als zweite Teilbedingung 24 der Auslösebedingung 9 geprüft, ob eine solche Bedieneingabe 25 erfasst wurde. Eine nutzerseitige Freigabe kann beispielsweise dann unterbleiben, wenn der Nutzer keine weitere Anpassung der Patientenposition wünscht, beispielsweise um schneller zu einer Bildgebung zukommen oder auch um eine spezifische Patientenpositionierung beizubehalten, die nutzerseitige gezielt gewählt ist.

Ist auch die zweite Teilbedingung 24 und somit die gesamte Auslösebedingung 9 erfüllt, so wird in Schritt S10 der Aktor 10 angesteuert, und die Lagereinrichtung 7 in die Sollposition 11 zu bringen.

Anschließend wird in Schritt S11 die Bildgebung wiederholt, um neue vorläufig Bilddaten 28 zu erfassen. Das Verfahren kann dann ab Schritt S2 wiederholt werden, um zu validieren, dass die auf Basis der neuen vorläufigen Bilddaten 28 ermittelte Merkmalsposition 8 hinreichend nahe an der Sollmerkmalsposition 29 ist. Ist dies nicht der Fall, so kann die Position der Lagereinrichtung 7, wie obig erläutert, erneut angepasst werden.

Ist für die vorläufigen Bilddaten 5 oder auch für die neuen vorläufigen Bilddaten 28 im Schritt S6 die erste Teilbedingung 22 der Auslösebedingung 9 hingegen nicht erfüllt, oder unterbleibt in Schritt S9 die Freigabe einer Bewegung der Lagereinrichtung 7 durch den Nutzer und ist somit die zweiten Teilbedingung 24 der Auslösebedingung 9 nicht erfüllt, so kann in Schritt S12 mit der Erfassung der Ergebnisbilddaten 4 fortgefahren werden, wobei insbesondere zunächst eine Planung dieser Bildgebung auf Basis der zuletzt erfassten vorläufigen Bilddaten 5 oder neuen vorläufigen Bilddaten 28, beispielsweise zur geeigneten Unterteilung des Untersuchungsvolumens in Messschichten, erfolgen kann.

In einer nicht gezeigten Weiterbildung des erläuterten Verfahrens könnte es beispielsweise möglich sein, dass dem Nutzer in Schritt S9 zusätzlich die Wahlmöglichkeit gegeben wird, die Positionierung und Bildgebung durch eine bestimmte Bedieneingabe vorübergehend zu unterbrechen oder abbrechen, beispielsweise um den Patienten 1 auf der Lagereinrichtung 7 umzulagern oder Ähnliches.

Eine Möglichkeit zur Bereitstellung des im Verfahren gemäß Fig. 2 genutzten Segmentierungsalgorithmus 13 wird im Folgenden mit Bezug auf Fig. 4 erläutert. Der Segmentierungsalgorithmus 13 wird hierbei durch ein Training eines Modells 21 durch ein überwachtes Maschinenlernen bereitgestellt.

Hierzu werden in Schritt S13 zunächst Eingangs-Trainingsdaten 32 bereitgestellt, die im Beispiel jeweils vorläufige Bilddaten umfassen, die den anatomischen Bereich, dessen Segmentierung erlernt werden soll, für verschiedene Patienten abbilden.

In Schritt S14 werden zusätzlich Ausgangs-Trainingsdaten 33 bereitgestellt, die ein jeweiliges Sollergebnis vorgeben, das bei Verarbeitung der jeweiligen Eingangs-Trainingsdaten 32 durch das trainierte Modell 21 resultieren soll. Die Ausgangs-Trainingsdaten 33 geben somit die Segmentierung der Eingangs-Trainingsdaten 32 vor und können beispielsweise durch eine manuelle Segmentierung der Eingangs-Trainingsdaten 32 durch Experten bereitgestellt werden. Eine solche Segmentierung kann beispielsweise vorgegeben werden, indem jedem Pixel oder Voxel der Eingangs-Trainingsdaten 32 eine Klasse zugeordnet wird. Da im oben erläuterten Beispiel Knochen segmentiert werden sollen, kann die jeweilige Klasse beispielsweise angeben, zu welchem Knochen der jeweilige Pixel beziehungsweise Voxel gehört beziehungsweise das er zu keinem Knochen gehört.

In Schritt S15 wird das Modells 21 dann, insbesondere iterativ, durch Maschinenlernen trainiert. Beispielhaft wird hierbei eine an sich bekannte Fehlerrückführung auf Basis eines Gradientenabstiegsverfahrens durchgeführt, bei der in jeder Iteration für einen jeweiligen Batch von Eingangs-Trainingsdaten 32 und zugehörigen Ausgangs-Trainingsdaten 33, eine Kostenfunktion ausgewertet wird. Die Kostenfunktion hängt hierbei von einer Abweichung eines jeweiligen Ergebnisses des Anwendens des Modells 21 im aktuellen Trainingszustand auf die Eingangs-Trainingsdaten 32 von den jeweiligen zugeordneten Ausgangs-Trainingsdaten 33 ab. Durch Ermittlung eines Gradienten dieser Kostenfunktion bezüglich der Parameter des Modells 21 wird dann ermittelt, wie stark und in welche Richtung die verschiedenen Parameter in der jeweiligen Iteration angepasst werden sollen.

Nach Abschluss des Trainings, beispielsweise nach einer vorgegebenen Zahl von Iterationen oder bei Erfüllung eines Konvergenzkriteriums, kann dann in Schritt S16 die aktuell vorliegende Parametrisierung des Modells 21 als trainiertes Modell 21 bereitgestellt werden, also beispielsweise aus einer Trainingsumgebung in einzelne ausgelieferte Computerprogramme, Verarbeitungseinrichtungen beziehungsweise medizinische Bildgebungseinrichtungen übertragen werden.

Ein zweckmäßiger Ansatz zur Segmentierung von Bilddaten durch ein durch Maschinenlernen trainiertes Modell ist eine Zuordnung von jeweiligen Klassen zu jeweiligen Pixeln oder Voxeln der Bilddaten durch das trainierte Modell und ein anschließendes zusammenfassen von Pixeln oder Voxeln der gleichen Klasse in einem jeweiligen Segment. Somit sind zu diesem Zweck Modelle geeignet, die eine Bild-zu-im Bild-Transformation implementieren.

Zur Bild-zu-Bild-Transformation und insbesondere, wie bereits im allgemeinen Teil der Beschreibung erläutert, zur Implementierung einer Segmentierung, sind neuronale Netze mit einer U-Net-Struktur beziehungsweise Kaskaden von solchen neuronalen Netzen, beispielsweise das bereits genannte nnU-Net, sehr gut geeignet.

Eine Beispiel für eine U-Net-Struktur ist in Fig. 5 dargestellt. Der Einfachheit halber wird im Beispiel davon ausgegangen, dass ein zweidimensionales Bild als Eingangsdaten verarbeitet wird und als Ergebnis wiederum ein zweidimensionales Bild resultiert, in dem der Bildwert der einzelnen Bildpunkte beispielsweise angibt, ob diese auf einer Kante liegen. Anhand solcher Ausgangsdaten können unmittelbar Kanten sowohl in x-Richtung als auch in y-Richtung erkannt werden. Das dargestellte Modell kann beispielsweise durch unüberwachtes lernen anhand von Trainingsdatensätzen trainiert werden, in denen Kanten beispielsweise durch geschultes Personal markiert sind. Im Rahmen des Trainings können eine Abweichung des Ergebnisses von einem Sollergebnisse minimiert werden, beispielsweise durch eine Fehlerrückführung, insbesondere durch ein Gradientenabstiegsverfahren.

Die Eingangsdaten für das trainierten Modells sind ein zweidimensionales medizinisches Bild mit 512x512 Bildpunkten, wobei jeder Bildpunkt einen Bildwert enthält. Das trainierte Modell besteht aus Faltungsschichten (gekennzeichnet durch durchgezogene, horizontale Pfeile), Pooling-Schichten (gekennzeichnet durch durchgezogene Pfeile, die nach unten zeigen) und Upsampling-Schichten (gekennzeichnet durch durchgezogene Pfeile, die nach oben zeigen), wobei die Anzahl der jeweiligen Knoten in den Kästen angegeben ist. Innerhalb der U-Netz-Struktur werden die Eingangsdaten zunächst heruntergetastet (Verkleinerung der Bilder und Erhöhung der Anzahl der Kanäle). Anschließend werden sie hochgetastet (Vergrößerung der Bilder und Verringerung der Anzahl der Kanäle), um ein transformiertes Bild zu erzeugen.

Alle Faltungsschichten L.1, L.2, L.4, L.5, L.7, L.8, L.10, L.11, L.13, L.14, L.16, L.17, L.19, L.20 außer der letzten Faltungsschicht L.21 verwenden 3x3-Faltungskernel mit einem Padding von 1, einer ReLU-Aktivierungsfunktion und eine Anzahl von Filtern beziehungsweise Faltungskernen, die der Anzahl der Kanäle der jeweiligen Schichten entspricht, wie sie in Fig. 5 angegeben ist. Die letzte Faltungsschicht L.22 verwendet einen 1x1-Faltungskernel ohne Padding und die ReLU-Aktivierungsfunktion.

Die Pooling-Schichten L.3, L.6, L.9 sind Max-Pooling-Schichten, die vier benachbarte Knoten durch nur einen Knoten ersetzen, wobei der Wert das Maximum der Werte der vier benachbarten Knoten ist. Die Upsampling-Schichten L.12, L.15, L.18 sind transponierte Faltungsschichten mit 3x3-Kernen und Stride 2, die die Anzahl der Knoten effektiv vervierfachen. Die gestrichelten horizontalen Pfeile entsprechen Verkettungsoperationen, bei denen der Ausgang einer Faltungsschicht L.2, L.5, L.8 des Abwärtssampling-Zweigs der U-Netz-Struktur als zusätzlicher Input für eine Faltungsschicht L.13, L.16, L.19 des Aufwärtssampling-Zweigs der U-Netz-Struktur verwendet wird. Diese zusätzlichen Eingangsdaten werden als zusätzliche Kanäle in der Eingangsknotenschicht für die Faltungsschicht L.13, L.16, L.19 des Upsampling-Zweiges behandelt.

## Patentansprüche

1. Computerimplementiertes Verfahren zur Positionierung eines Patienten (1) bezüglich wenigstens einer Komponente (2) einer medizinischen Bildgebungseinrichtung (3) zur Erfassung von Ergebnisbilddaten (4), umfassend die Schritte:
- Erfassen vorläufiger Bilddaten (5), die wenigstens ein vorgegebenes anatomisches Merkmal (6) des Patienten (1) zumindest zum Teil abbilden, während der Patient (1) oder ein das anatomische Merkmal (6) umfassender Abschnitt des Patienten (1) durch eine Lagereinrichtung (7) der medizinischen Bildgebungseirichtung (3) gelagert ist,
- Ermitteln einer Merkmalsposition (8) des vorgegebenen anatomischen Merkmals (6) in den vorläufigen Bilddaten (5), und, stets oder bei Erfüllung einer von der Merkmalsposition (8) abhängigen Auslösebedingung (9),
- Ansteuern eines Aktors (10) der Bildgebungseinrichtung (3) zur Bewegung der Lagereinrichtung (7) in eine auf Basis der Merkmalsposition (8) ermittelte Sollposition (11) und/oder Ausgabe eines die Sollposition (11) betreffenden Hinweises (12) an einen Nutzer der Bildgebungseinrichtung (1).

2. Computerimplementiertes Verfahren nach Anspruch 1,
**dadurch gekennzeichnet,**
**dass** durch einen Segmentierungsalgorithmus (13) eine Segmentierung (16) wenigstens eines Knochens (14, 15) des Patienten (1) in den vorläufigen Bilddaten (5) erfolgt, wobei die Merkmalsposition (8) in Abhängigkeit der Segmentierung (16) ermittelt wird.

3. Computerimplementiertes Verfahren nach Anspruch 2,
**dadurch gekennzeichnet,**
**dass** für zwei der Knochen (14, 15), die benachbart zueinander angeordnet sind, ein jeweiliges den jeweiligen Knochen (14, 15) abbildendes Segment (17, 18) der vorläufige Bilddaten (5) ermittelt wird, wobei als das anatomische Merkmal (6), dessen Merkmalsposition (8) bestimmt wird, ein zwischen diesen Segmenten (17, 18) angeordneter Zwischenbereich (19) oder eine Grenzlinie oder eine Grenzfläche (20) zwischen diesen Segmenten (17, 18) verwendet wird.

4. Computerimplementiertes Verfahren nach Anspruch 2 oder 3,
**dadurch gekennzeichnet,**
**dass** als der Segmentierungsalgorithmus (13) ein durch Maschinenlernen trainiertes Modell (21) verwendet wird.

5. Computerimplementiertes Verfahren nach Anspruch 4,
**dadurch gekennzeichnet,**
**dass** das durch Maschinenlernen trainierte Modell (21) ein neuronales Netz mit einer U-Net-Struktur ist oder umfasst.

6. Computerimplementiertes Verfahren nach einem der vorangehenden Ansprüche,
**dadurch gekennzeichnet,**
**dass** bei Erfüllung einer die Sollposition (11) auswertenden ersten Teilbedingung (22) der Auslösebedingung (9) zunächst eine Freigabeanforderung (23) an den Nutzer ausgegeben wird, wobei die Ansteuerung des Aktors (10) zur Bewegung der Lagereinrichtung (7) in die Sollposition (11) nur bei Erfüllung einer zweiten Teilbedingung (24) der Auslösebedingung (9) erfolgt, wobei die zweite Teilbedingung (24) erfüllt wird oder ausschließlichen dann erfüllbar ist, wenn eine eine Freigabe der Bewegung indizierenden Bedieneingabe (25) des Nutzers an der medizinischen Bildgebungseinrichtung (3) und/oder an einer in einer Kommunikationsverbindung mit der medizinischen Bildgebungseinrichtung (3) stehenden Freigabeeinrichtung (26) erfasst wird.

7. Computerimplementiertes Verfahren nach einem der vorangehenden Ansprüche,
**dadurch gekennzeichnet,**
**dass** stets oder bei Erfüllung der Auslösebedingung (9) und/oder der ersten Teilbedingung (22) in Abhängigkeit der Merkmalsposition (8) eine von mehreren vorgegebenen Erläuterungsinformationen (27) ausgewählt und als Teil des die Sollposition (11) betreffenden Hinweises (12) und/oder der Freigabeanforderung (23) ausgegeben wird.

8. Computerimplementiertes Verfahren nach einem der vorangehenden Ansprüche,
**dadurch gekennzeichnet,**
**dass** die Erfassung der vorläufiger Bilddaten (5) nach der Ansteuerung des Aktors (10) zur Bewegung der Lagereinrichtung (7) in die Sollposition automatisch zur Ermittlung neuer vorläufiger Bilddaten (28) wiederholt wird.

9. Computerimplementiertes Verfahren nach einem der vorangehenden Ansprüche,
**dadurch gekennzeichnet,**
**dass** als medizinischen Bildgebungseinrichtung (3) eine Magnetresonanzeinrichtung verwendet wird.

10. Computerimplementiertes Verfahren zum Bereitstellen eines durch Maschinenlernen trainierten Modells (21), das dazu eingerichtet ist, in dem computerimplementierten Verfahren nach einem der vorangehenden Ansprüche als der Segmentierungsalgorithmus (13) und/oder zur Ermittlung der Merkmalsposition (8) verwendet zu werden, umfassend die Schritte:
- Empfangen von Eingangs-Trainingsdaten (32),
- Empfangen von Ausgangs-Trainingsdaten (33), wobei die Ausgangs-Trainingsdaten (33) eine jeweilige Klasse der Pixel oder Voxel der Eingangs-Trainingsdaten (32) vorgeben und/oder eine Segmentierung der Eingangs-Trainingsdaten (32) beschreiben,
- Trainieren des Modells (21) durch Maschinenlernen auf der Grundlage der Eingangs-Trainingsdaten (32) und der Ausgangs-Trainingsdaten (33), und
- Bereitstellen des durch Maschinenlernen trainierten Modells (21).

11. Verarbeitungseinrichtung, **dadurch gekennzeichnet, dass** die sie zur Durchführung des computerimplementierten Verfahrens nach einem der vorangehenden Ansprüche eingerichtet ist.

12. Magnetresonanzeinrichtung, **dadurch gekennzeichnet, dass** sie eine Verarbeitungseinrichtung (34) nach Anspruch 11 umfasst.

13. Computerprogramm mit Anweisungen, die dazu eingerichtet sind, bei einer Ausführung auf einer Datenverarbeitungsvorrichtung (35) das computerimplementierte Verfahren nach einem der Ansprüche 1 bis 10 durchzuführen.

14. Datenträger, **dadurch gekennzeichnet, dass** er das Computerprogramm (36) nach Anspruch 13 umfasst.
